(19) Europäisches Patentamt
European Patent Office
Office européen des brevets

(11) Numéro de publication: **0 384 796 A1**

(12) **DEMANDE DE BREVET EUROPEEN**

(21) Numéro de dépôt: **90400244.1**

(22) Date de dépôt: **30.01.90**

(51) Int. Cl.5: **C07D 311/40, C07D 311/32, A61K 7/00, A61K 31/35**

(30) Priorité: **15.02.89 FR 8901947**

(43) Date de publication de la demande: **29.08.90 Bulletin 90/35**

(84) Etats contractants désignés: **AT BE CH DE DK ES FR GB GR IT LI LU NL SE**

(71) Demandeur: **SOCIETE CIVILE POUR L' EXPANSION DE LA RECHERCHE EN PHYTOCHIMIE APPLIQUEE**
**Domaine de Valois - Martillac**
**F-33650 La Brede(FR)**

(72) Inventeur: **Masquelier, Jack, Parc des Tourelles Les Magnolias**
**Rue Sainte-Elisabeth**
**F-33200 Bordeaux Cauderan(FR)**

(74) Mandataire: **Cournarie, Michèle et al**
**Office Blétry 2, boulevard de Strasbourg**
**F-75010 Paris(FR)**

(54) **Procédé de préparation d'extraits polyphénoliques de type flavane-3-ol purifiés et extraits obtenus.**

(57) Procédé de préparation d'extraits polyphénoliques de type flavane-3-ol purifiés ("pyonogénols") à partir de diverses matières premières végétales, caractérisé en ce qu'il comprend les étapes consistant à épuiser la matière végétale brute tout d'abord par l'eau, à filtrer le mélange, à centrifuger le filtrat après séjour au froid, à épuiser le surnageant par l'acétate d'éthyle, à séparer la phase organique contenant l'extrait, à distiller cette phase sous pression réduite pour récupérer l'acétate d'éthyle, à reprendre par l'eau le résidu aqueux et à lyophiliser la solution aqueuse obtenue, ce qui fournit l'extrait recherché sous forme d'une poudre entièrement soluble dans l'eau.

Utilisation de l'extrait obtenu comme substance active, à effet capteur sur les radicaux libres, dans des produits pharmaceutiques, diététiques et cosmétiques.

EP 0 384 796 A1

# Procédé de préparation d'extraits polyphénoliques de type flavane-3-ol purifiés et extraits obtenus

L'invention concerne un procédé de préparation d'extraits polyphénoliques de type flavane-3-ol et les extraits obtenus qui trouvent des applications en pharmacie, en diététique et en cosmétologie.

Le terme "pyonogénol" (étymologiquement : qui engendre des produits condensés) a été attribué en 1979 à une famille de polyphénols végétaux jusqu'alors englobés dans le groupe disparate des flavonoïdes, (cf. J. Masquelier et al dans Internat. J. Vit.Nutr.Res., 1979, 49, 307-311).

Le système cyclique fondamental des pyonogénols est celui du 2-phénylchromanne (I)

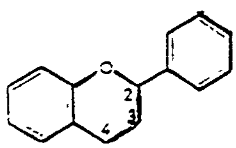

(I)

sur lequel sont généralement plusieurs groupements hydroxy en particulier en position 3 et 4.

C'est le plus souvent par l'intermédiaire d'une condensation avec élimination d'$H_2O$ entre le groupement hydroxy en C-4 d'une molécule et l'atome d'hydrogène en C-8 d'une autre molécule que se forment facilement les oligomères et les polymères supérieurs, un exemple de dimère (le procyanidol) ayant la formule suivante II

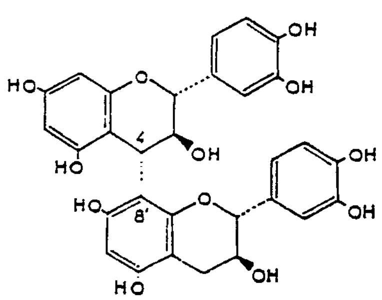

Les pyonogénols forment un ensemble homogène et autonome dont le caractère chimique essentiel est une propension à la condensation. Leur intérêt biologique réside principalement dans leur intense pouvoir capteur envers les radicaux libres oxygénés (J. Masquelier, Bull. O.I.V. 1988, 689-690, 554-578) qui leur ouvre de nombreuses applications en pharmacie, en diététique et en cosmétologie.

Bien que largement répartis dans le règne végétal, les pyonogénols ne s'y trouvent pas à des concentrations élevées et sont entourés de divers polyphénols qui ne présentent pas d'intérêt sur le plan biologique recherché et sont en outre instables voire même toxiques et qu'il faut donc éliminer de l'extrait de pyonogénols. De ce fait, les techniques d'extraction antérieures mettent en oeuvre divers mélanges de solvants et nécessitent des opérations complexes de fractionnement et de purification, avec des rendements médiocres et à des coûts de production importants.

En effet, les plantes à pyonogénols qui sont des leucoanthocyanes incolores sont généralement des plantes à tanins, en raison d'une voie biogénétique commune. Or, ces tanins constituent une fraction à éliminer, en raison de leur peu d'intérêt pharmacologique, de leur instabilité et des incompatibilités biologiques qu'ils provoquent à cause de leur masse moléculaire élevée (fixation irréversible aux protéines sanguines par exemple).

Dans l'industrie, l'élimination des tanins met en jeu le relargage par les électrolytes. C'est ainsi que de nombreux procédés d'épuisement d'un matériel végétal comportent une phase de précipitation des tanins condensés indésirables à l'aide du chlorure de sodium à saturation (cf. par exemple FR-A-1 427 100). Cela entraîne le rejet d'énormes volumes de solutions salines, dont on ne peut se débarrasser sans polluer gravement l'environnement. Tout procédé permettant d'éliminer ces tanins sans le recours à ce salage constitue donc un progrès primordial sous l'angle industriel. En outre, l'absence de sel permet éventuellement de récupérer le tanin comme sous-produit.

Dautres documents décrivent l'extraction de composés différents des pyonogénols.

FR-A-2 276 059 décrit l'extraction par l'eau d'anthocyanes. Ceux-ci sont des pigments rouges extractibles par l'eau mais seulement en présence d'anhydride sulfureux, et ne sont en solution alcoolique que sous forme de chlorure.

FR-A-2 276 060 décrit l'extraction par l'eau et par l'acétate d'éthyle d'un extrait flavonique. Cet extrait se caractérise par l'absence de leucoanthocyanes (page 2, lignes 3-4).

Par ailleurs, en raison même de leur nature, les pyonogénols existent chez les plantes sous divers degrés de condensation : monomères, dimères, oligomères, polymères, que l'on trouve le plus souvent côte à côte dans la matière première végétale. Etant donné que les effets pharmacologiques ne sont pas uniformément répartis entre ces différentes formes de condensation, il importe que le procédé d'extraction privilégie les fractions possédant au maximum la propriété recherchée. Par exemple, s'agissant des effets antiradicalaires, il faut obtenir en priorité les oligomères (degré de condensation égal à 2, 3 et 4), puisque l'expérience démontre que leur pouvoir capteur est nettement plus élevé

que calui des autres fractions. Une action antivirale conduira, en revanche, à sélectionner les polymères.

Il est donc utile de pouvoir préparer à partir d'une matière première végétale un extrait riche en pyonogénols et suffisamment purifié pour servir de matière première pour la confection de médicaments, de produits diététiques et de cosmétiques.

La solubilité des pyonogénols dans l'eau est remarquable. Sauf pour les hauts polymères, qui ont peu d'intérêt au point de vue pharmacologique, l'eau s'avère donc le solvant extractif de choix, ses principaux avantages étant :
- l'extraction d'un large éventail de pyonogénols
- l'exclusion des hauts polymères
- son coût faible par rapport à celui des solvants organiques.

La présente invention fournit un tel procédé, qui comprend les étapes consistant à épuiser la matière végétale brute tout d'abord par l'eau, à filtrer le mélange, à centrifuger le filtrat après séjour au froid, à épuiser le surnageant par l'acétate d'éthyle, à séparer la phase organique contenant l'extrait, à distiller cette phase sous pression réduite pour récupérer l'acétate d'éthyle, à reprendre par l'eau le résidu aqueux et à lyophiliser la solution aqueuse obtenue, ce qui fournit l'extrait recherché, sous forme d'une poudre entièrement soluble dans l'eau.

De façon plus détaillée, le procédé comporte les étapes suivantes :

1. Epuisement de la matière première végétale par l'eau

2. Extraction de la solution aqueuse ainsi obtenue, directement par un solvant organique non miscible à l'eau mais envers lequel les pyonogénols manifestent un coefficient de partage le plus élevé possible. L'acétate d'éthyle remplit cette condition. En outre, il s'avère sélectif vis-à-vis des pyonogénols peu condensés : contrairement à ce que l'on pensait généralement, il ne dissout pas les tanins. La saturation par le chlorure de sodium de la solution aqueuse, qui semblait être le préalable essential à l'élimination des tanins, peut donc être supprimée dans ce nouveau procédé, ce qui constitue une innovation aux conséquences pratiques importantes sur le plan industriel.

3. L'acétate d'éthyle, séparé de la phase aqueuse, est évaporé sous pression réduite, jusqu'à l'obtention d'un résidu aqueux privé de tout reste de solvant organique. Cette solution est lyophilisée et le produit obtenu constitue l'extrait destiné aux usages diététiques et cosmétologiques.

Le titre en pyonogénols de l'extrait obtenu est déterminé par spectrophotométrie.

Pour obtenir un produit davantage purifié, par exemple pour usage pharmaceutique, l'extrait lyophilisé peut subir une purification complémentaire consistant à le dissoudre dans de l'acétate d'éthyle et à le précipiter de cette solution par un solvant organique chloré tel que le dichlorométhane. On peut renouveler une seconde fois cette opération.

Les différentes étapes peuvent être mises en oeuvre de la façon suivante.

La première opération (extraction par l'eau) peut revêtir de multiples modalités où interviennent principalement quatre paramètres : volumes, température, durée, pH.

- le rapport V/P (volume d'eau/poids de matière brute) peut varier dans une large fourchette, selon que la matière végétale est sèche ou fraîche, compacte ou divisée, mouillable ou hydrofuge. Il est bien évident que l'extraction de feuilles vertes exigera moins d'eau qu'une écorce sèche. Chaque type de matière brute constitue un cas d'espèce.

- La température de l'eau peut, elle aussi, faire l'objet de multiples ajustements, depuis la simple macération à froid (+4°C), à la température du laboratoire, ou à des températures variant de +20° à 100°C.

L'eau à l'ébullition (décoction) et la vapeur d'eau sous pression (percolation) sont également utilisables.

Le choix de la température idoine résulte d'essais préliminaires assortis du dosage global des pyonogénols extraits.

- La durée de l'extraction par l'eau influe, dans chaque cas, sur le rendement, et doit être ajustée selon les résultats du dosage. Plus la température est élevée, plus le traitement est court, mais les pyonogénols ne supportent pas un long séjour à température élevée : une extraction à +80° est, à cet égard, moins agressive qu'une décoction à l'ébullition.

- Le pH : le contenu cellulaire végétal est en général situé à des pH inférieurs à 7. Par ailleurs, les pyonogénols supportent mal un séjour en milieu alcalin. On doit donc veiller à ce que le pH de l'eau soit ≦pH 6 (ce qu'il est en général) au cours de l'extraction. En outre, il peut être utile d'ajouter à l'eau une substance réductrice, telle que le sulfite acide de sodium, à raison de 0,5g par litre par exemple, pour éviter l'oxydation des pyonogénols en cours d'extraction. De même, il faut priver l'eau de traces de fer ou de cuivre qui activent l'autooxydation des polyphénols. L'eau distillée ou désionisée est à conseiller.

Pour la seconde opération (épuisement à l'acetate d'éthyle de la solution aqueuse précédemment obtenue), diverses modalités d'exécution peuvent aussi s'appliquer :
- Technique de l'épuisement : l'acétate d'éthyle n'est pas miscible à l'eau, mais à son contact, il s'en sature. L'épuisement, qui est effectué à la température ambiante, peut se faire :
par simple agitation des deux liquides et décantation, soit spontanée, soit accélérée par centrifugation;
par épuisement continu dans un extracteur du type HAGEN, ou par la technique à contre-courant.

- Le rapport $V_1/V_2$, (volume de la solution aqueuse/volume d'acétate d'éthyle), varie selon le mode d'épuisement, la richesse du végétal en pyonogénols, les impératifs industriels de rendement.

- La distillation de l'acétate d'éthyle à l'issue de l'épuisement met en oeuvre toute technique permettant d'éliminer entièrement le solvant sans lui faire subir une température trop élevée. On y parvient en créant un vide partiel, de telle sorte que le solvant distille sans dépasser la température de +50 °C.; un atomiseur permettant de respecter cette norme peut fort bien être employé.

Des exemples de matières végétales pouvant être traitées par le procédé suivant l'invention sont les suivants sans que cette liste soit limitative :
pépins de raisins; sommités fleuries d'aubépine; bractées de tilleul; écorce de pin des Landes; feuilles de noisetier; feuilles de ginkgo biloba; écorce de quinquina; racine de rhubarbe etc .

Pour apprécier le rendement de l'extraction, en suivre les variations selon les modifications apportées et titrer l'extrait obtenu en pyonogénols, on a adopté une technique spectrophotométrique de dosage. En effet, les pyonogénols présentent, en lumière ultra-violette, un spectre d'absorption dont le maximum est voisin de 280 nm, et le minimum voisin de 256 nm.

On compare le résultat obtenu avec celui que donne le produit de référence, soit l'extrait séché à 100 °C jusqu'à poids constant.

Le coefficient d'extinction à 280 nm d'une solution dans l'alcool à 95° de 4mg de produit sec pour 100ml est de :

$$E_{1cm}^{1p100} = 160.$$

Tous les extraits obtenus selon le procédé sont identifiables par la réaction de BATE-SMITH. A 10ml d'une solution aqueuse de l'extrait à 1%, on ajoute 2ml d'acide chlorhydrique pur et l'on chauffe le mélange au bain-marie bouillant (100 °C). Une intense coloration rouge se forme en quelques minutes. Elle est due à une transformation des pyonogénols en dérivés des chlorures d'oxonium correspondants (anthocyanes). A ce titre, la réaction est spécifique et permet donc la caractéristique des produits obtenus.

On décrit ci-après, à titre illustratif et nullement limitatif, deux exemples de mise en oeuvre du procédé de l'invention.

Exemple 1

200g de pépins entiers de raisin (Vitis vinifera) sont soumis pendant 2h à une infusion dans 2l d'eau distillée contenant 0,5g/l de sulfite acide de sodium, chauffée à 80 °C sous agitation constante. Le mélange est filtré à chaud. Le filtrat (1600ml) séjourne ensuite une nuit à + 4 °C, puis on le centrifuge. Un culot de matières insolubles est abandonné. Le surnageant fait l'objet d'une mesure d'absorption à 280nm. On l'épuise alors par l'acétate d'éthyle (600ml) dans un extracteur liquide-liquide à contre-courant, jusqu'à ce que l'absorption de la phase aqueuse mesurée à 280nm soit réduite au 1/10$^{e}$ de sa valeur initiale.

L'acétate d'éthyle (500ml) est distillé sous pression réduite à une température inférieure à 50 °C. Il abandonne un résidu aqueux que l'on amène à 90ml par addition d'eau. La lyophilisation de cette dernière solution fournit un produit sec qui constitue l'extrait destiné aux emplois diététiques et cosmétologiques.

Rendement : 9,55g par kg de pépins secs. A titre de comparaison, l'extraction de ces pépins selon une technique conventionnelle incluant un relargage par le chlorure de sodium ne livre que 4,78g d'extrait.

Exemple 2.

200g d'écorce de pin des Landes (Pinus maritima) grossièrement concassée subissent un traitement identique à celui de l'exemple 1. Le rendement en extrait pour usage diététique et cosmétologique est de 6,07g par kg. Les techniques anciennes avec relargage au chlorure de sodium appliquées à la même matière première ont un rendement de 3,0g par kg. En vue d'un usage pharmaceutique, l'extrait lyophilisé (5g) est mis en solution dans l'acétate d'éthyle (100ml). On ajoute à cette solution 200ml de dichlorométhane. Un volumineux précipité de pyonogénols se forme aussitôt. On le recueille par filtration sur verre fritté. Le filtrat, qui entraîne les impuretés, est distillé pour récupérer les solvants.

Les extraits obtenus selon le procédé de l'invention sont des produits pulvérulents, entièrement solubles dans l'eau. Leur richesse en pyonogénols les destine en particulier aux emplois reposant sur un effet capteur envers les radicaux libres oxygénés (cf. J. MASQUELIER, Congrès de la Société de Nutrition et de Diététique de langue française, Bordeaux, 26 et 27 septembre 1986 et UCHIDA,S et al., Med. Sci. Res., 1987, 15, 831-832 qui montrent que l'effet antiradicalaire des pyonogénols surpasse de loin celui de tous les capteurs naturels déjà connus).

L'usage cosmétologique (crèmes, gels, solutions, laits corporels, aérosols, sels de bain, capsules ...) et l'usage diététiques à titre de complément alimentaire (en nature sous forme de poudres, solutions, tablettes, gélules, granulés ... ou incorporés à des produits de régime déjà existants) constituent donc les emplois majeurs des extraits obtenus selon le procédé.

La préparation de médicaments est également possible, en particulier pour des indications dermatologiques.

Le test au dithranol (J. MASQUELIER, inédit) montre en effet que les pyonogénols, très actifs in vitro sur les réactions radicalaires, manifestent le même pouvoir dans l'organisme humain. Il consiste à déposer sur la peau, en deux endroits distincts de la face interne de l'avant-bras, 10 microlitres d'une solution de dithranol à 2‰ dans le chloroforme, sur une aire circulaire de 15mm de diamètre. On laisse évaporer le solvant. Sur l'une des deux surfaces, on dépose 10 microlitres d'une solution alcoolique à 1% de l'extrait à tester. Après 24h, la surface témoin est le siège d'une forte réaction inflammatoire due à la production de radicaux libres oxygénés (HINNEN, M.J. et coll., The Lancet, nov. 17, 1984, p. 1129-1130). La surface traitée par les pyonogénols montre une réaction très fortement atténuée, ou même une absence d'inflammation, ce qui prouve bien que l'extrait selon le procédé exerce son pouvoir capteur in vivo sur l'organisme de l'homme. Un tel résultat n'a jamais pu être démontré objectivement avec les produits préconisés jusqu'à présent, pour lesquels seuls des tests in vitro fondent les propriétés antiradicalaires.

Les radicaux libres sont incriminés en tant que facteurs pathogéniques du vieillissement, de la sénescence et de nombreuses affections médicales et chirurgicales. On sait également (NORDMANN, R., et coll., Bull. Acad. Ntle. Md., 1985, 169, 1201-1206) qu'ils sont impliqués dans les effets toxiques hépatiques et nerveux de l'alcoolisme. Les extraits titrés en pyonogénols selon le procédé permettent d'envisager une prévention de ces effets, en raison de leur pouvoir capteur élevé vis-à-vis des radicaux libres oxygénés.

## Revendications

1. Procédé de préparation d'extraits polyphénoliques de type flavane-3-ol purifiés ("pyonogénols") à partir de diverses matières premières végétales, caractérisé en ce qu'il comprend les étapes consistant à épuiser la matière végétale brute tout d'abord par l'eau, à filtrer le mélange, à centrifuger le filtrat après séjour au froid, à épuiser le surnageant par l'acétate d'éthyle, à séparer la phase organique contenant l'extrait, à distiller cette phase sous pression réduite pour récupérer l'acétate d'éthyle, à reprendre par l'eau le residu aqueux et à lyophiliser la solution aqueuse obtenue, ce qui fournit l'extrait recherché sous forme d'une poudre entièrement soluble dans l'eau.

2. Procédé suivant la revendication 1, caractérisé en ce que l'on effectue l'épuisement par l'eau à une température comprise entre 4°C et 100°C et avec une eau distillée ou désionisée dont le pH est inférieur ou égal à 6, en ce que l'on effectue l'épuisement par l'acétate d'éthyle à la température ambiante, et en ce que l'on distille la phase organique, pour récupérer l'acétate d'éthyle, sous un vide tel que la température de distillation ne dépasse pas 50°C.

3. Procédé suivant la revendication 1 ou 2, caractérisé en ce que l'eau utilisée pour l'épuisement par l'eau est additionnée d'une substance réductrice.

4. Procédé suivant la revendication 3, caractérisé en ce que la substance réductrice est le sulfite de sodium.

5. Procédé suivant l'une quelconque des revendications 1 à 4, caractérisé en ce que la matière première végétale est choisie parmi les matières végétales suivantes : pépins de raisins; sommites fleuries d'aubépine; bractées de tilleul; écorce de pin des Landes; feuilles de noisetier; feuilles de ginkgo biloba; écorce de quinquina; racine de rhubarbe.

6. Procédé suivant l'une quelconque des revendications 1 à 5, caractérisé en ce que la matière première végétale est constituée par des pépins entiers de raisins, en ce que l'épuisement par l'eau est une infusion effectuée à 80°C sous agitation constante et suivie d'une filtration à chaud, l'eau employée étant de l'eau distillée ou désionisée, ayant un pH inférieur à 6, et additionnée de 0,5g/l de sulfite acide de sodium, en ce que l'épuisement par l'acétate d'éthyle est effectué dans un extracteur à contre-courant à la température ambiante, et en ce que la distillation sous pression réduite des phases organiques surnageantes réunies est effectuée à une temperature inférieure à 50°C.

7. Extrait polyphénolique de type flavane-3-ol purifié obtenu par le procédé suivant l'une quelconque des revendications 1 à 6.

8. Application de l'extrait suivant la revendication 7 comme constituant de produits cosmétiques, de produits diététiques et de médicaments.

Office européen des brevets

# RAPPORT DE RECHERCHE EUROPEENNE

Numero de la demande

EP 90 40 0244

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FR-A-2 276 059 (INSTITUT NATIONAL DE LA RECHERCHE AGRONOMIQUE)<br>* page 4, exemple; revendications 1,2,4 *<br>--- | 1-3 | C 07 D 311/40<br>C 07 D 311/32<br>A 61 K 7/00<br>A 61 K 31/35 |
| Y | FR-A-2 276 060 (SOCIETE CORTICAL)<br>* page 1, lignes 16-37 *<br>--- | 1-3 | |
| A | CHEMICAL ABSTRACTS<br>tome 79, no. 4, 30 juillet 1973, abrégé no. 20475t, Columbus, Ohio, US; A.E. SOSNIN: "Effect of additions of chemical reagents on extraction of water-extractive substances from pine bark" & Tr. Arkhangel'sk. Lesotekh. Inst. 1972, no. 32, pages 35-39<br>--- | 1,3,4 | |
| A | US-E- 25 135 (E. KURTH et al.)<br>* page 1, colonne 2, lignes 1-15 *<br>--- | 1,4 | |
| A | FR-A-1 427 100 (SOCIETE CIVILE DE RECHERCHE PHARMACEUTIQUE ET THERAPEUTIQUE)<br>* revendication 1, exemple *<br>--- | 1 | **DOMAINES TECHNIQUES RECHERCHES (Int. Cl.5)** |
| A | CH-A- 626 359 (INVERNI DELLA BEFFA)<br>* exemple, revendications 1-3 *<br>----- | 1,5 | C 07 D 311/00<br>A 61 K 31/00 |

Le présent rapport a été établi pour toutes les revendications

| Lieu de la recherche | Date d'achèvement de la recherche | Examinateur |
|---|---|---|
| BERLIN | 16-05-1990 | PROBERT C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)